# EUROPEAN PATENT SPECIFICATION

(11) **EP 4 397 657 B1**
(45) Date of publication and mention of the grant of the patent: **11.11.2026**
(21) Application number: 23740544.4
(22) Date of filing: 17.01.2023
(51) Int. Cl.: C07D 251/30, C07D 251/34

(54) **COMPOUND, METHOD FOR PREPARING SAME, AND SINGLE MOLECULE AND OLIGOMER DERIVED FROM SAME**
VERBINDUNG, VERFAHREN ZUR HERSTELLUNG DAVON UND DAVON ABGELEITETES EINZELMOLEKÜL UND OLIGOMER
COMPOSÉ, SON PROCÉDÉ DE PRÉPARATION, ET MOLÉCULE UNIQUE ET OLIGOMÈRE DÉRIVÉS DE CELUI-CI

(30) Priority: 17.01.2022 KR 20220006316
(43) Date of publication of application: 10.07.2024
(73) Proprietor: LG Chem, Ltd., Yeongdeungpo-gu Seoul 07336 (KR)
(72) Inventor: PARK, Beomsu, Daejeon 34122 (KR); JUNG, Jihye, Daejeon 34122 (KR); KIM, Hyun Cheol, Daejeon 34122 (KR)
(74) Representative: Goddar, Heinz J.
(86) International application number: PCT/KR2023/000790
(87) International publication number: WO 2023/136701

(56) References cited:
- EP-A1- 3 498 756
- EP-A1- 4 008 741
- WO-A1-2021/024964
- JP-A- 2006 036 973
- JP-A- 2012 111 856
- JP-A- H02 129 257
- KR-A- 20190 042 921
- KR-A- 20190 134 328

## Description

### [Technical Field]

The present specification relates to a compound, a method for preparing the same, and an oligomer derived from the compound.

### [Background Art]

Triallyl isocyanurate is useful as a cross-linking agent having excellent heat resistance and chemical resistance, and is expected to be used in a wide range of fields such as electronic materials, liquid crystals, semiconductors, and solar cells. For example, in printed circuit boards, that is, plate-like or film-like parts that constitute electronic circuits by fixing a large number of electronic parts such as integrated circuits, resistors, and condensers to a surface thereof and connecting the parts through wiring, triallyl isocyanurate is used as a sealing material for preventing penetration of materials such as liquids or gases into the parts.

However, triallyl isocyanurate has high relative permittivity and high dissipation factor, so that research has been continuously conducted to reduce them. WO2021/024964 A1 and EP 3498756 A1 disclose compounds based on a triallyl isocyanurate structure having a polyperfluoroalkylether substituent.

### [Detailed Description of the Invention]

### [Technical Problem]

The present specification provides a compound, a method for preparing the same, and an oligomer derived from the compound.

### [Technical Solution]

The present specification provides a compound of the following Chemical Formula 1.

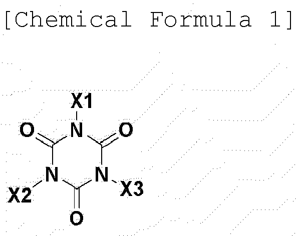

In Chemical Formula 1,
at least one of X1 to X3 is 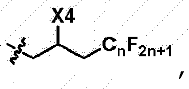 and the others are an allyl group,
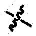 is a moiety bonded to Chemical Formula 1, and wherein the 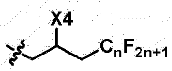 is any one of

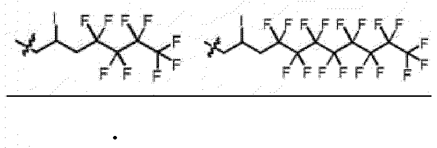

The present specification provides a method for preparing a compound of the following Chemical Formula 1, the method including: (s1) putting triallyl isocyanurate, CₙF₂ₙ₊₁X4, a base, and a solvent into a container and stirring the resulting mixture under nitrogen gas; and (s2) adding a radical initiator thereto and stirring the resulting mixture.

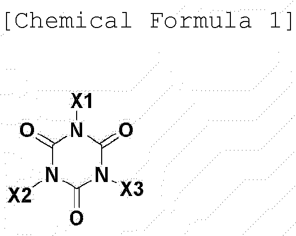

In Chemical Formula 1,
at least one of X1 to X3 is 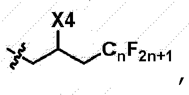 and the others are an allyl group,
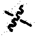 is a moiety bonded to Chemical Formula 1, and wherein the 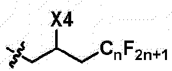 is any one of

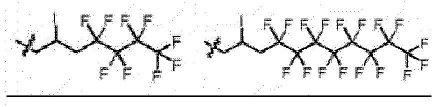

Further, the present specification provides an oligomer including a monomer derived from the compound. Further embodiments are disclosed in the dependent claims.

### [Advantageous Effects]

The compound according to an exemplary embodiment of the present specification, and an oligomer derived therefrom include a good release group and a structure capable of being cross-linked, so that various derivatives can be prepared.

The compound according to an exemplary embodiment of the present specification can be used as a polyfunctional monomer to exhibit the effects of achieving low refractive index, low relative permittivity, low surface energy and low dissipation factor.

In the method for preparing a compound according to an exemplary embodiment of the present specification, the reaction is performed under mild conditions with minimal side reactions, so that the yield of the compound of Chemical Formula 1, which is a target compound, is increased.

### [Brief Description of Drawings]

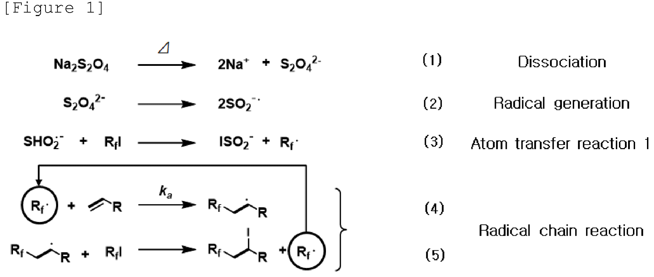

FIG. 1 is a view describing the mechanism of the method for preparing the compound of Chemical Formula 1.

### [Best Mode]

Hereinafter, the present specification will be described in more detail.

The present specification provides a compound of the following Chemical Formula 1.

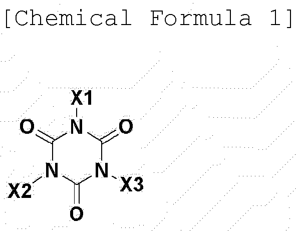

In Chemical Formula 1,
at least one of X1 to X3 is 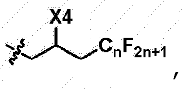 and the others are an allyl group,
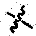 is a moiety bonded to Chemical Formula 1, and wherein the 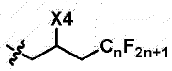 is any one of

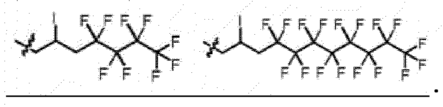

The compound of Chemical Formula 1 according to an exemplary embodiment of the present specification includes a perfluoroalkyl group including fluorine having the effects of achieving low relative permittivity and low dissipation factor, and is used as a polyfunctional monomer as a triallyl isocyanurate derivative that serves as a cross-linking agent, so that it is possible to exhibit the effects of achieving low relative permittivity, low surface energy and low dissipation factor.

According to an exemplary embodiment of the present specification, any one of X1 to X3 is 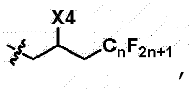 and the others are an allyl group.

According to an exemplary embodiment of the present specification, any two of X1 to X3 are 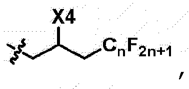 and the other is an allyl group.

According to an exemplary embodiment of the present specification, X1 to X3 are each

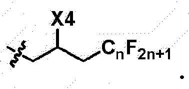

According to an exemplary embodiment of the present specification, compounds with various molecular weights may be prepared because the chain length of a perfluoroalkyl group is easily adjusted. In addition, when a perfluoroalkyl group is included, it is possible to exhibit effects of achieving low refractive index, low relative permittivity, low surface energy and low dissipation factor.

According to an exemplary embodiment of the present specification, Chemical Formula 1 is any one selected from the following compounds.

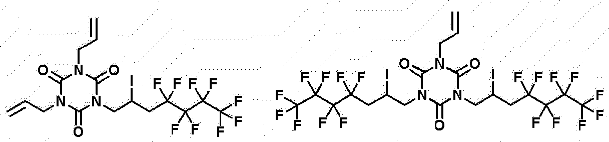

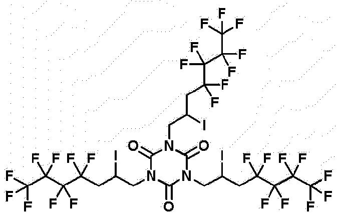

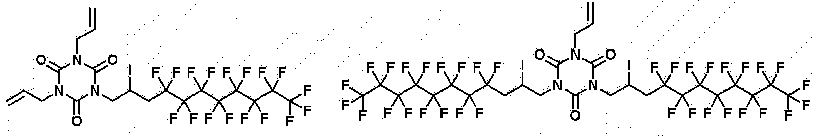

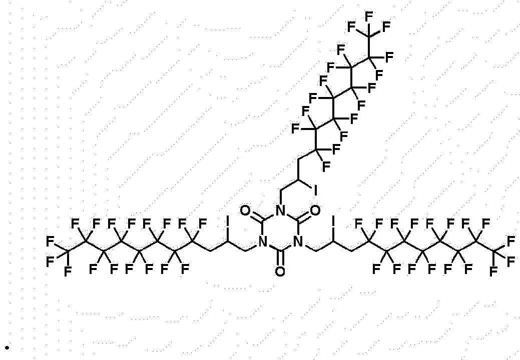

The present specification provides a method for preparing a compound of the following Chemical Formula 1, the method including: (s1) introducing triallyl isocyanurate, CₙF₂ₙ₊₁X4, a base and a solvent and stirring the resulting mixture under nitrogen gas; and (s2) adding a radical initiator thereto and stirring the resulting mixture.

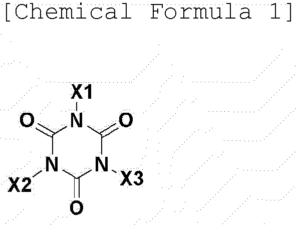

In Chemical Formula 1,
at least one of X1 to X3 is 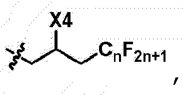 and the others are an allyl group,
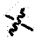 is a moiety bonded to Chemical Formula 1, and wherein the 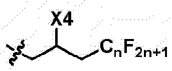 is any one of

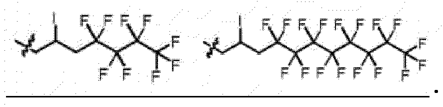

According to an exemplary embodiment of the present specification, the reaction temperature in Step (s1) is 0°C to room temperature. When the above reaction is performed within the above reaction temperature range, the reaction conditions are not tricky but mild, and side reactions that appear as the temperature rises are suppressed, so that the yield of the compound of Chemical Formula 1, which is a final target, is increased. In addition, various temperature ranges may be set according to the range of n and the molecular weight of the compound of Chemical Formula 1 in Step (s1).

In the present specification, room temperature means 20±5°C under atmospheric pressure.

The base according to an exemplary embodiment of the present specification may be potassium carbonate (K₂CO₃), sodium hydrogen carbonate (NaHCO₃), potassium hydrogen carbonate (KHCO₃) or any mixture thereof, but is not limited thereto, and a salt used in the related art may be used.

According to an exemplary embodiment of the present specification, the solvent is an organic solvent.

According to an exemplary embodiment of the present specification, the solvent is selected from among hexane, heptane, toluene, benzene, acetonitrile, dichloromethane, dichloroethane, trichloroethane, chloroform, dichloroform, nitromethane, dibromomethane, cyclopentanone, cyclohexanone, fluorobenzene, bromobenzene, chlorobenzene, xylene, mesitylene, ethylacetate or any mixture thereof, but is not limited thereto, and an organic solvent used in the related art may be used.

According to an exemplary embodiment of the present specification, the radical initiator is a water-soluble initiator.

According to an exemplary embodiment of the present specification, the radical initiator is any one of potassium persulfate (KPS), ammonium persulfate (APS), potassium hydrosulfite, sodium hydrosulfite, sodium persulfate, or hydrogen peroxide.

According to an exemplary embodiment of the present specification, the reaction temperature in Step (s2) is 0°C. When Step (s2) is performed at the above reaction temperature, side reactions are suppressed, so that the yield of Chemical Formula 1, which is a final target, is increased.

The mechanism of the preparation method according to an exemplary embodiment of the present specification is described in the following FIG. 1. (1) The radical initiator is dissociated under basic conditions, (2) radicals are generated, and (3) the atom transfer reaction occurs. The hydrogen of the initiator is dissociated in (3) and bonded to a halogen group (an iodine group) bonded to perfluoroalkyl, and the perfluoroalkyl becomes a radical. Thereafter, by the radical chain reaction of (4) and (5), (4) the radicalized perfluoroalkyl group and the allyl group of the triallyl isocyanurate are bonded to form a radical, and (5) the halogen group (the iodine group) bonded to the perfluoroalkyl is bonded to the radical to produce the compound of Chemical Formula 1.

In FIG. 1, *Kₐ* is the addition rate constant of a radical to substrate (R_{f}).

According to an exemplary embodiment of the present specification, a step of obtaining the compound of Chemical Formula 1 is further included after Step (s2).

The obtaining step of the above according to an exemplary embodiment of the present specification includes:
(s31) a quench and layer separation step;
(s32) a drying step;
(s33) a filtration step;
(s34) a concentration step; and
(s35) an obtaining step.

According to an exemplary embodiment of the present specification, Step (s31) (quench and layer separation step) is a step of terminating the reaction by adding a reaction termination material, and separating the layers.

According to an exemplary embodiment of the present specification, as the reaction termination material of Step (s31), distilled water, an aqueous ammonium chloride solution, an aqueous sodium hydrogen carbonate solution, an aqueous KF (saturated) solution, an aqueous HCl solution, an aqueous NaCl (saturated) solution, chloroform, dichloromethane, and ethyl acetate may be used, but the reaction termination material is not limited thereto. Preferably, as the reaction termination material, an aqueous ammonium chloride solution, an aqueous sodium hydrogen carbonate solution, an aqueous NaCl solution, and ethyl acetate are used.

In Step (s31), the order in which the reaction termination and layer separation are performed is not limited to a specific order. As an exemplary embodiment, reaction termination and layer separation may be simultaneously performed, and as another exemplary embodiment, the reaction is terminated by adding a reaction termination material, and then layers may be separated. However, the progression order is not limited to the above-described example.

Since the radical initiator remaining after the reaction is water-soluble, the radical initiator may be dissolved in water and removed in Step (s31).

According to an exemplary embodiment of the present specification, Step (s32) (drying step) is performed by adding a desiccant to any one layer of the material layer-separated in Step (s31). For example, when chloroform exemplified above is used as the reaction termination material, Step (s32) is performed by adding a desiccant to a chloroform layer which is a lower layer, that is, an organic layer.

Examples of the desiccant include magnesium sulfate, and sodium sulfate, but are not limited thereto. According to a preferred exemplary embodiment, the desiccant is magnesium sulfate.

In the present specification, the desiccant is added in a sufficient amount required for typical drying in the art.

According to an exemplary embodiment of the present specification, Step (s33) (filtration step) may be performed by a method known in the art. As a representative example, it is possible to use a method of inhaling air using an inhaler and filtering impurities including a desiccant, which are separated from a target product with a filter, or a method of removing other impurities using a solvent that does not dissolve a target product, but dissolves other impurities by utilizing the difference in solubility in a specific solvent.

According to an exemplary embodiment of the present specification, Step (s34) (concentration step) may be performed by a method known in the art. The step may be performed by evaporating the solvent using a vacuum rotary evaporator as a representative example, but the representative example is not limited thereto.

According to an exemplary embodiment of the present specification, additional filtration and drying steps may be added after Step (s34).

It is possible to include a drying step before the obtaining step (s35), and the drying step is a step of drying the compound of Chemical Formula 1. The drying step is different from Step (s32) and may be performed by a method known in the art, representative examples thereof include vacuum oven drying, spray drying, and flash drying, and preferably, the compound of Chemical Formula 1 may be dried by vacuum oven drying.

According to an exemplary embodiment of the present specification, Step (s35) (obtaining step) is a step of obtaining the compound of Chemical Formula 1 which is concentrated (dried) in Step (s34).

According to an exemplary embodiment of the present specification, in the obtaining step, Steps (s32) to (s33) may be performed three or more times, if necessary.

According to an exemplary embodiment of the present specification, provided is a mixture including two or more of the compounds.

According to another exemplary embodiment of the present specification, two or more compounds included in the mixture are the same as or different from each other. That is, it means that the two or more compounds are the same as or different from each other while having the structure of Chemical Formula 1.

According to still another exemplary embodiment of the present specification, the mixture may further include a compound different from the compound of Chemical Formula 1.

According to the present specification, provided is an oligomer including a monomer derived from the compound.

Those skilled in the art may understand the term "monomer" described in the present specification as a state in which a compound is polymerized to be linked to the main chain of the oligomer.

In the present specification, for example, the "monomer derived from the compound of Chemical Formula **1"** is a repeating unit that constitutes the main chain in the polymer, and means that an allyl group of the compound of Chemical Formula 1 may form a radical to become a monomer, and a halogen group may be used as an electron donor to introduce a monomer or end group which makes up the main chain of other oligomers.

Furthermore, radicals generated by the release of the halogen group in the structure may also react with other allyl group-containing oligomers or the above monomers.

According to an exemplary embodiment of the present specification, the oligomers may have the following structures, but is not limited thereto. Oligomers with various structures may be shown depending on the range of n in Chemical Formula 1, that is, n from 1 to 20.

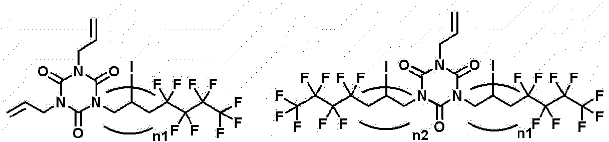

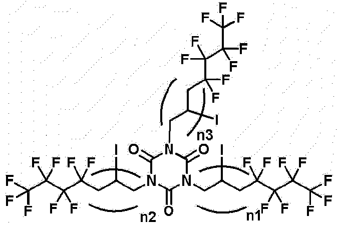

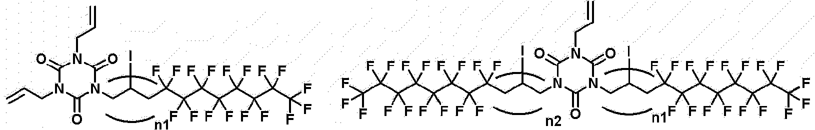

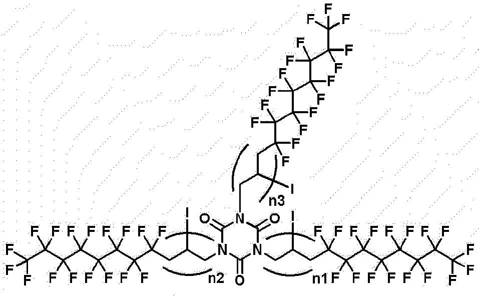

In the structures,
n1 to n3 are each an integer of 2 or higher.

According to an exemplary embodiment of the present specification, n1 to n3 are each an integer from 2 to 10,000, preferably an integer from 2 to 5,000, more preferably an integer from 2 to 1000, and even more preferably an integer from 2 to 100.

The compound according to an exemplary embodiment of the present specification, and an oligomer derived therefrom include a good release group and a structure capable of being cross-linked, so that various derivatives can be prepared.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below in detail. The Examples of the present specification are provided to explain the present specification more completely to a person with ordinary skill in the art.

### Example 1.

After 9.9 g of triallyl isocyanurate, 0.101 g of NaHCO₃ and 20.7 mL of nonafluoro-1-iodobutane were dissolved in 100 mL of water and 50 mL of acetonitrile in a 250 mL two-neck round-bottom flask and N₂ bubbling was performed at room temperature for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 44 g of a product which was derived from perfluorobutyl iodide, and the weight ratios of Compound 1-2, Compound 1-3 and Oligomer 1 are shown in the following Table 1 by determining the detailed composition of the product by ¹H-NMR.

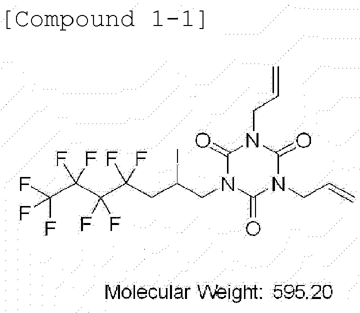

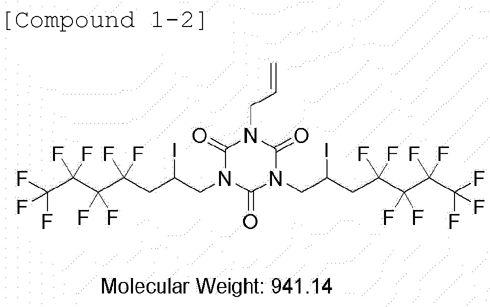

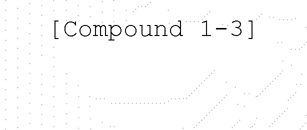

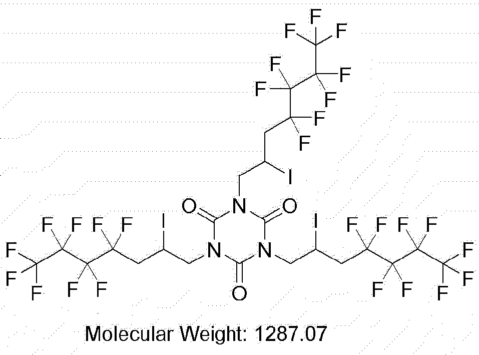

**[Table 1]**

| Structure | Compound 1-2 | Compound 1-3 | Oligomer 1 |
|---|---|---|---|
| Weight ratio | 80 to 90 | 1 to 10 | 1 to 10 |

### Example 2.

After 9.9 g of triallyl isocyanurate, 0.101 g of NaHCO₃ and 10.3 mL of nonafluoro-1-iodobutane were dissolved in 100 mL of water and 50 mL of acetonitrile in a 250 mL two-neck round-bottom flask and N₂ bubbling was performed at room temperature for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 22 g of a product which was derived from perfluorobutyl iodide, and the weight ratios of Compound 1-1, Compound 1-2 and Oligomer 1 are shown in the following Table 2 by determining the detailed composition of the product by ¹H-NMR.

**[Table 2]**

| Structure | Compound 1-1 | Compound 1-2 | Oligomer 1 |
|---|---|---|---|
| Weight ratio | 70 to 80 | 10 to 20 | 1 to 10 |

Oligomer 1 of Examples 1 and 2 is derived from any one or more of Compounds 1-1 to 1-3, and means a product whose molecular weight exceeds that of Compound 1-3, whose molecular weight is the maximum unit as a single molecule.

### Example 3.

After 9.97g of triallyl isocyanurate, 0.101 g of NaHCO₃ and 31.7 mL of heptadecafluoro-n-octyl iodide were dissolved in 100 mL of water and 40 mL of acetonitrile in a 250 mL two-neck round-bottom flask and N₂ bubbling was performed at 0°C for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 53 g of a product which was derived from perfluorooctyl iodide, and the weight ratios of Compound 2-2, Compound 2-3 and Oligomer 2 are shown in the following Table 3 by determining the detailed composition of the product by ¹H-NMR.

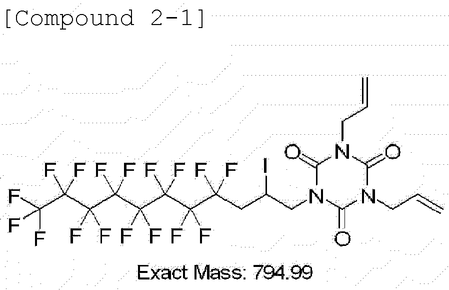

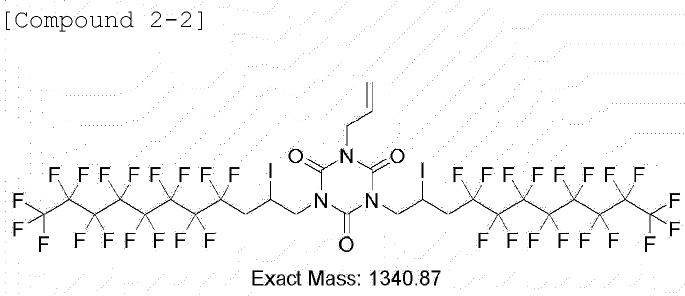

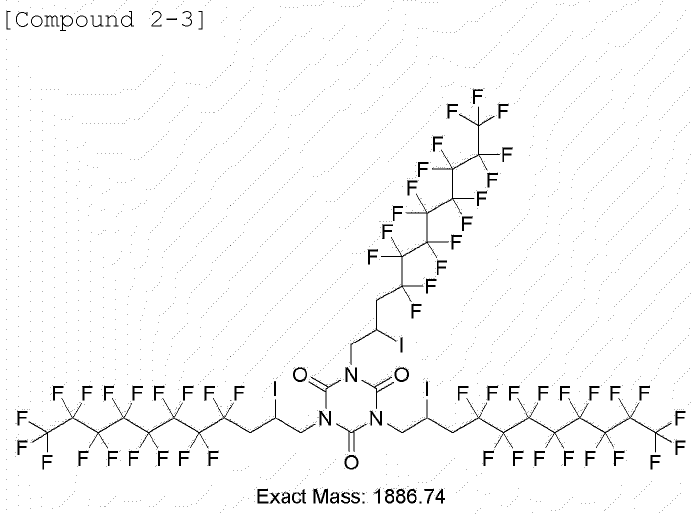

**[Table 3]**

| Structure | Compound 2-2 | Compound 2-3 | Oligomer 2 |
|---|---|---|---|
| Weight ratio | 10 to 20 | 80 to 90 | 1 to 10 |

### Example 4.

After 9.97g of triallyl isocyanurate, 0.101 g of NaHCO₃ and 15.8 mL of heptadecafluoro-n-octyl iodide were dissolved in 100 mL of water and 40 mL of acetonitrile in a 250 mL two-neck round-bottom flask and N₂ bubbling was performed at 0°C for 30 minutes, Na₂S₂O₄ was added thereto in a powder state, and then the resulting mixture was stirred at 0°C for 17 hours. Thereafter, after the aqueous layer was extracted three times with 200 mL of chloroform, the organic layer was washed with water, and the washed organic layer was dried over 10 g of MgSO₄. Thereafter, filtration was performed and the solvent was evaporated to obtain 36 g of a product from which perfluorooctyl iodide was derived, and the weight ratios of Compound 2-1, Compound 2-2 and Oligomer 2 are shown in the following Table 4 by determining the detailed composition of the product by ¹H-NMR.

**[Table 4]**

| Structure | Compound 2-1 | Compound 2-2 | Oligomer 2 |
|---|---|---|---|
| Weight ratio | 40 to 50 | 40 to 50 | 1 to 10 |

Oligomer 2 of Examples 3 and 4 is derived from any one or more of Compounds 2-1 to 2-3, and means a product whose molecular weight exceeds that of Compound 2-3, whose molecular weight is the maximum unit as a single molecule.

The compound of Chemical Formula 1 prepared in Tables 1 to 4 includes a perfluoroalkyl group including fluorine having the effects of achieving low relative permittivity and low dissipation factor, and is used as a polyfunctional monomer as a triallyl isocyanurate derivative that serves as a cross-linking agent, so that it is possible to exhibit the effects of achieving low refractive index, low relative permittivity, low surface energy and low dissipation factor. The compound of Chemical Formula 1 may be used for various uses.

The compound of Chemical Formula 1 may be used as a material for circuit boards, the relative permittivity is predicted to be about Dₖ = 2.5 (Reference = 2.6 or higher) in a specific composition, and through the aforementioned result, it can be predicted that there is an effect of lowering the relative permittivity.

- Reference: Triallyl isocyanurate

## Claims

1. A compound of the following Chemical Formula 1: 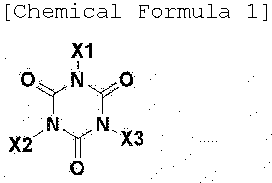
wherein, in Chemical Formula 1,
at least one of X1 to X3 is 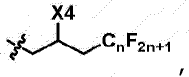 and the others are an allyl group,
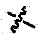 is a moiety bonded to Chemical Formula 1, and wherein the 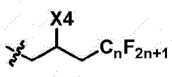 is any one of

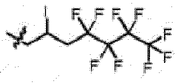

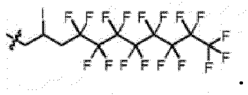

2. The compound of claim 1, wherein Chemical Formula 1 is any one selected from the following compounds:

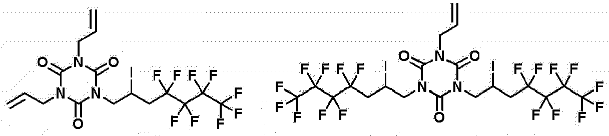

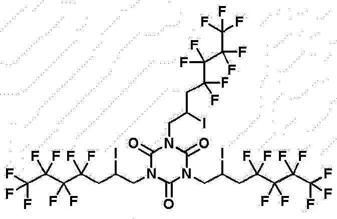

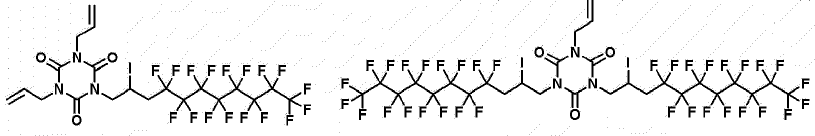

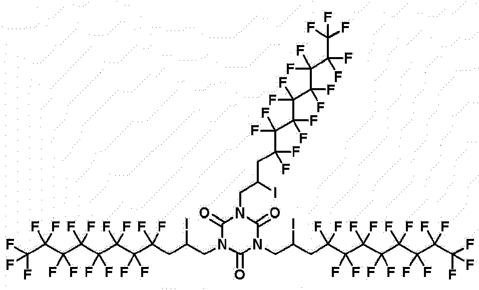

3. A method for preparing a compound of the following Chemical Formula 1, the method comprising: (s1) putting triallyl isocyanurate, CₙF₂ₙ₊₁X4, a base, and a solvent into a container and stirring the resulting mixture under nitrogen gas; and
(s2) adding a radical initiator thereto and stirring the resulting mixture: 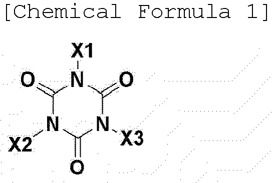
wherein, in Chemical Formula 1,
at least one of X1 to X3 is 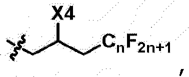 and the others are an allyl group,
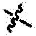 is a moiety bonded to Chemical Formula 1, and
wherein the 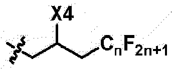 is any one of

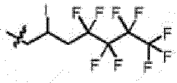

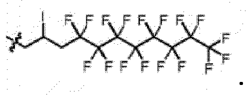

4. An oligomer comprising a monomer derived from the compound of any one of claims 1 and 2.

## Patentansprüche

1. Verbindung der folgenden chemischen Formel 1: 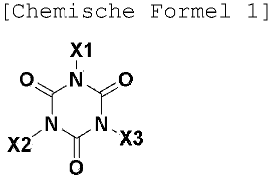
wobei in der chemischen Formel 1
mindestens eines von X1 bis X3 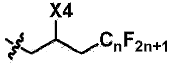 ist und die anderen eine Allylgruppe sind,
eine Einheit ist, die an die chemische Formel 1 gebunden ist, und
wobei das 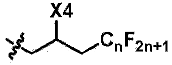 eines von 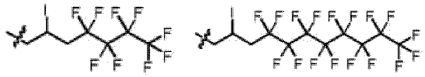 ist.

2. Verbindung nach Anspruch 1, wobei die chemische Formel 1 eine ist, die aus den folgenden Verbindungen ausgewählt ist:

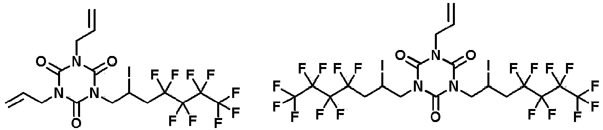

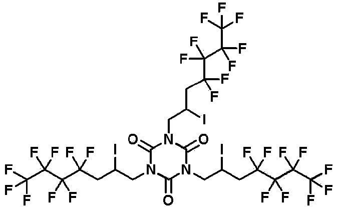

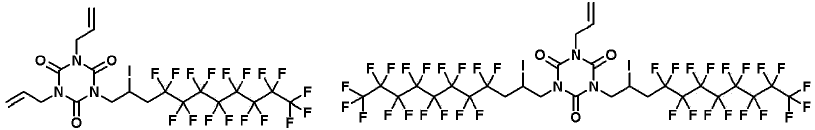

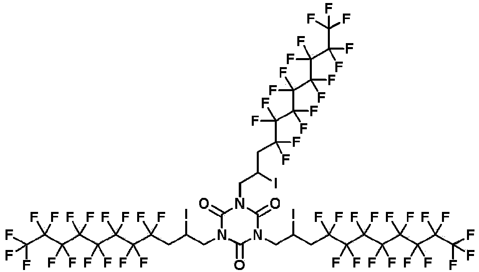

3. Verfahren zur Herstellung einer Verbindung der folgenden chemischen Formel 1, wobei das Verfahren umfasst: (s1) Einbringen von Triallylisocyanurat, CₙF₂ₙ₊₁X4, einer Base und eines Lösungsmittels in einen Behälter und Rühren der resultierenden Mischung unter Stickstoffgas; und
(s2) Zugeben eines Radikalinitiators dazu und Rühren der resultierenden Mischung: 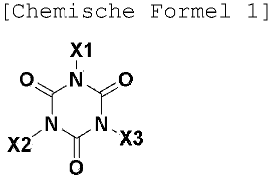
wobei in der chemischen Formel 1
mindestens eines von X1 bis X3 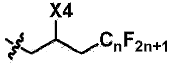 ist und die anderen eine Allylgruppe sind,
eine Einheit ist, die an die chemische Formel 1 gebunden ist, und
wobei das 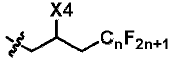 eines von 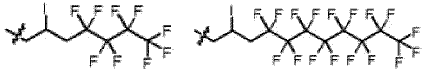 ist.

4. Oligomer, umfassend ein Monomer, das von der Verbindung nach einem der Ansprüche 1 und 2 abgeleitet ist.

## Revendications

1. Composé de la Formule chimique 1 ci-après : 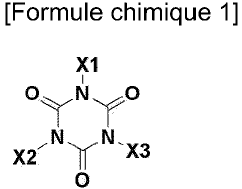 dans la Formule chimique 1,
au moins un parmi X1 à X3 est 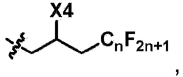 et les autres sont un groupe allyle,
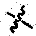 est une fraction liée à la Formule chimique 1, et dans lequel 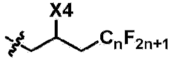 est l'un quelconque parmi

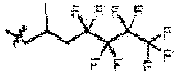

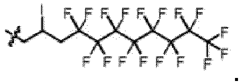

2. Composé selon la revendication 1, dans lequel la Formule chimique 1 est sélectionnée parmi les composés suivants :

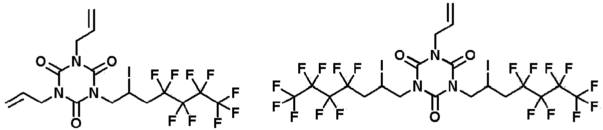

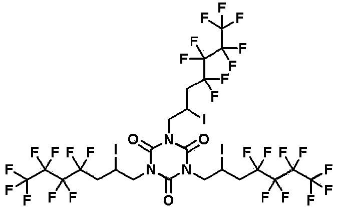

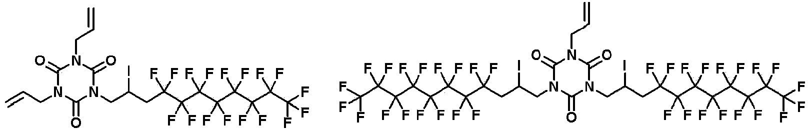

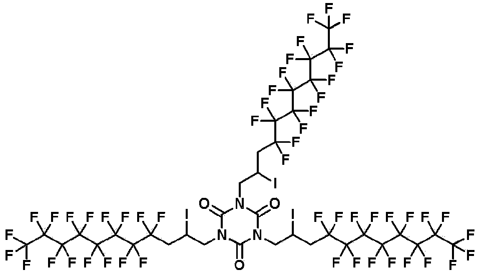

3. Procédé de préparation d'un composé de la Formule chimique 1 ci-après, le procédé consistant à : (s1) placer un isocyanurate de triallyle, CₙF₂ₙ₊₁X4, une base et un solvant dans un contenant et agiter le mélange résultant sous azote gazeux; et
(s2) ajouter un initiateur radical et agiter le mélange résultant : 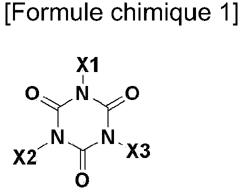
dans lequel, dans la Formule chimique 1,
au moins un parmi X1 à X3 est 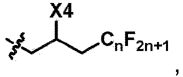 et les autres sont un groupe allyle,
est une fraction liée à la Formule chimique 1, et
dans lequel 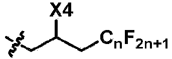 est un parmi

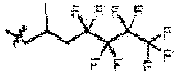

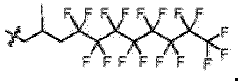

4. Oligomère comprenant un monomère dérivé du composé selon l'une des revendications 1 et 2.
